# EUROPEAN PATENT SPECIFICATION

(11) **EP 1 766 072 B1**
(45) Date of publication and mention of the grant of the patent: **04.08.2010**
(21) Application number: 05758622.4
(22) Date of filing: 03.06.2005
(51) Int. Cl.: C07H 21/02, C07H 21/04, C12P 19/34, C12Q 1/68

(54) **HYBRIDIZATION OF PNA PROBES IN ALCOHOL SOLUTIONS**
HYBRIDISIERUNG VON PNA-SONDEN IN ALKOHOLLÖSUNGEN
HYBRIDATION DE SONDES PNA DANS DES SOLUTIONS ALCOOLIQUES

(30) Priority: 03.06.2004 US 577409 P
(43) Date of publication of application: 28.03.2007
(73) Proprietor: AdvanDx, Inc., Woburn MA 01801-7476 (US)
(72) Inventor: FIANDACA, Mark, Princeton, MA 01541 (US); OLIVEIRA, Kenneth, Sudbury, MA 01776 (US); STENDER, Henrik, DK-2820 Gentoffe (DK)
(74) Representative: Stevens, Ian Edward
(86) International application number: PCT/US2005/019570
(87) International publication number: WO 2005/121373

(56) References cited:
- WO-A-00/66788
- WO-A-02/095052
- US-A- 4 124 449
- US-A- 5 225 326
- US-A- 5 225 326
- US-A- 5 256 571
- US-A1- 2003 108 872
- US-A1- 2004 115 692
- US-B1- 6 750 039
- US-B2- 6 280 946
- US-B2- 6 753 421
- CHANG ET AL.: 'Bacterial meningitis in infants: the epidemiology, clinical features, and prognostic factors' BRAIN & DEVELOPMENT vol. 26, no. 3, April 2004, pages 168 - 175, XP003012823

## Description

### Field of the Invention

This invention is related to the field of probe based nucleic acid sequence detection, analysis and quantification. More specifically, this invention relates to the use of PNA probes in aqueous alcohol solutions for diagnostic applications. In one aspect, the invention enables sample preparation and analysis to be performed as a single step.

### Background

Nucleic acid hybridization is a fundamental physiochemical process, central to the understanding of molecular biology. Probe-based assays use hybridization for the detection, quantitation and analysis of nucleic acids. Nucleic acid probes have long been used to analyze samples from a variety of sources for the presence of nucleic acids, as well as to examine clinical conditions of interest in single cells and tissues. More recently, peptide nucleic acid probes have become the preferred reagents for hybridization assays.

Despite its name, Peptide Nucleic Acid (PNA) is neither a peptide, a nucleic acid, nor is it even an acid. PNA is a non-naturally occurring polyamide that can hybridize to nucleic acids (DNA and RNA) with sequence specificity (See: U.S. Pat. No. 5,539,082) and Egholm et al., Nature 365:566-568 (1993)) according to Watson-Crick base paring rules. However, whereas nucleic acids are biological materials that play a central role in the life of living species as agents of genetic transmission and expression, PNA is a recently developed totally artificial molecule, conceived in the minds of chemists and made using synthetic organic chemistry. PNA also differs structurally from nucleic acids. Although both can employ common nucleobases (A, C, G, T, and U), the backbones of these molecules are structurally diverse. The backbones of RNA and DNA are composed of repeating phosphodiester ribose and 2-deoxyribose units. In contrast, the backbones of the most common PNAs are composed of (aminoethyl)-glycine subunits. Additionally, in PNA the nucleobases are connected to the backbone by an additional methylene carbonyl moiety. PNA is therefore not an acid and therefore contains no charged acidic groups such as those present in DNA and RNA.

Aqueous alcohol solutions, such as PreservCyt and SurePath sample transfer reagents (see US525671) are commonly used in diagnostic settings where samples need to be obtained from the patient at one time and preserved for analysis at a later time. The use of alcohol containing solutions for this purpose has become commonplace in diagnostic settings. There are several benefits to alcohol solutions which make them ideal for sample preservation. Alcohols such as ethanol, methanol, and isopropanol (2-propanol) are miscible in water. Alcohol solutions maintain cellular nucleic acids (DNA and RNA) and preserve the integrity of cellular organelles. Alcohol solutions can be used to sterilize a sample, killing potentially infectious organisms, while maintaining them structurally for later detection. Alcohols are highly volatile, and thus can be easily removed from a surface by evaporation. This characteristic allows a simple method for applying a liquid mixture sample to a surface, then removing all of the liquid and leaving the sample on the surface. Alcohol solutions have excellent qualities for preservation of samples such as biopsies, cervical swabs, tissue sections, and other specimens at ambient temperature or lower for significant periods of time.

Alcohols have the effect of lowering the Tm of DNA hybrids. The amount of DNA hybrid destabilization caused by alcohol is dependant on several factors including the type of alcohol, the length, and sequence of the DNAs, and the concentration and type of salts present. For example, in the case of ethanol, the Tm of a given DNA-DNA hybrid is reduced linearly at the approximate rate of 10% decrease for every 10% of ethanol in solution (Srivastava et al, J. Biochem. Biophys. (1979) 16, 427-431). At a certain point, again depending on the factors described above, DNA will form aggregates and precipitate out of solution. From what we know of the effect of alcohol solutions on the stability of DNA hybrids, it would be surprising and indeed novel to propose introduction of alcohols to hybridization reactions for practical applications. Alcohols used for fixation and/or preservation of specimens are therefore routinely removed either by evaporation or by one or more wash steps prior to performing hybridization reactions with PNA probes (Perry-O'Keefe et, J. Micro. Meth. (47) 281-292, 2001, Oliveira et., J. Clin. Microbiol 40:247-251 (2002)).

WO 02/095052 describes PNA probes, probe sets and methods and kits pertaining to the detection of Candida yeast.

US 5,225,326 discloses an *in situ* hybridization assay for detecting as few as 1 to 5 copies of target nucleic acid per cell.

It would be desirable to have methods and kits using alcohol solutions as hybridization solutions. Especially desirable would be to have an In situ hybridization method for specimens fixed in an alcohol solution which uses the same alcohol solution as a hybridization solution, therefore providing a simple and user friendly procedure. Ideally, the PNA probe is already in the alcohol solution prior to fixation of the specimen such that fixation and hybridization can be performed as a single step.

### SUMMARY OF THE INVENTION

The present invention provides methods and kits for hybridizing a PNA probe to a target sequence as set out in the appended claims. In one aspect, the invention features compositions in which at least one PNA probe is provided in combination with an aqueous alcohol solution that serves, for instance, as a specimen fixing and probe hybridization medium. The invention has a wide spectrum of important applications including use in pathogen detection assays.

It has been found that in contrast to most DNA sequences, a PNA probe can specifically hybridize to its complementary nucleic acid target in the presence of an aqueous alcohol solution. It is thus an object of the invention to take advantage of this observation by providing a composition that includes at least one PNA probe and the solution. Preferred solutions include one or a combination of lower alcohols as needed to suit a particular invention application. A distinct advantage of the invention is that preferred compositions are intended to serve, for instance, as a specimen fixing agent. That is, the compositions can not only fix the specimen but also, can provide an efficient hybridization medium for specifically binding the PNA probe to the target sequence. Methods of the invention will generally use one composition to analyze the target sequence, however for some applications more than one may be helpful such as one, two or three of such compositions. Nearly any suitable target sequence can be analyzed according to the invention including those embodied in fixed, partially fixed or unfixed samples of interest.

There is acknowledgement in the field that aqueous alcohol solutions reduce the Tm of DNA:DNA hybrids by decreasing the hydration of counter ions such as Na+, and K+. These ions are generally thought to assist formation of the hybrids. However in the presence of alcohol, hydration is thought to decrease and reduce association with the DNA backbone. This phenomenon is thought to destabilize the double helical structure of the hybrid. While not wishing to be bound to any theory, it is known that most PNA probes have a relatively higher tolerance for the decrease in counter ion concentration than DNA. This is believed to help the probe bind target in the presence of alcohol. The present invention takes advantage of this important theoretical observation.

Practice of the invention is well suited for a range of applications including those in which analysis of at least one target nucleic acid in a sample is desired. In one embodiment of the method, cells are contacted with the composition under conditions conducive for specifically binding the PNA probe to target nucleic acid therein. Cells are then incubated with the composition under conditions that form a specific binding complex between the PNA probe and any target nucleic acid in the sample. Any detected (bound) PNA probe is taken to be indicative of the presence of the target nucleic acid in the cells. Such methods can be performed with a suitable control sample(s), for instance, those in which no target nucleic acid is provided as well as those in which a known quantity of the target is provided. Examples of suitable controls are discussed and exemplified below.

More specifically, it has been found that preferred invention compositions can penetrate the membrane of cells fixed in the aqueous alcohol solution and subsequently hybridize in situ to complementary nucleic acid targets in those cells. Significantly, and in this embodiment, the same aqueous alcohol solution is used as both a fixation and hybridization solution or medium. These invention features provide several advantages including assisting practice of in situ hybridization assays that use one or a combination of detection formats. Examples of such formats include those that directly or indirectly rely on fluorescence in situ hybridization.

Practice of the invention can help avoid the time and expense of adding probe to a specimen after it is fixed. That is, the invention can be used, In one embodiment, to provide for essentially simultaneous fixation and hybridization of the PNA probe to target sequence. In this invention example, the need to use conventional assay steps involving the removal, exchange, inactivation and/or addition of chemicals needed for either fixation or hybridization is greatly reduced and often avoided.

As discussed below, preferred aqueous alcohol solutions provide excellent stringency for hybridization with the PNA probes, such that fluorescence in situ hybridization assays, for example, can be performed without a stringent post-hybridization wash step to remove unhybridized PNA probe(s) prior to measuring the fluorescence intensity within the cells.

It is an object to provide an invention that is flexible and readily adaptable to suit an intended use. The PNA probe is combined with the aqueous alcohol solution directly. This invention embodiment is especially helpful in settings were rapid detection formats are useful. The invention methods are further adaptable and compatible with use of multiple aqueous alcohol solutions and /or PNA probes depending on the particular application required. The methods of the invention are not tied to any particular order of combining the PNA probe, aqueous alcohol solution and sample of interest so long as intended results are achieved.

The present invention provides further uses and advantages. For instance, it has been found that PNA linear beacons can maintain their self-reporting capabilities in aqueous alcohol solutions. This observation was surprising given acknowledged denaturing characteristics of many alcohols. In embodiments in which the invention composition includes at least one self-reporting PNA Linear beacon, such a composition can be used as part of a relatively simple fluorescence in situ hybridization assay. Typically, the assay will have few or no manual steps, such as centrifugation, filtration or washing that are often used to remove, replace or add components of the assay. In one embodiment of such an assay, an incubation step at defined temperature is essentially the only step needed other than addition of the specimen to the aqueous alcohol solution containing PNA probe(s) and the final measurement of the fluorescence intensity within the cells. Thus, practice of these invention embodiments will save the user time and expense in the analysis of desired target sequences.

The invention is also well suited as a means to combine probe-based analysis with morphological analysis of cytological specimens, such as PAP smears. In this context, the PNA probe(s) are added to fixation solutions, such as PreservCyt, SurePath or equivalent solutions, for analysis by in situ hybridization. Following hybridization the cells are transferred onto microscope slides optionally involving a filtration step, such as but not limited to ThinPreps, for further analysis and/or measurement of the fluorescent intensity or other detectable signal within the cells. Further analysis may comprise signal amplification or other detection technologies.

The invention provides still further uses and advantages. For instance, and in one embodiment, it is adaptable to a wide range of techniques and instruments for measuring the detectable signal within cells. Exemplary but not limiting detection systems include microscopes, fluorescent microscopes, slide scanners, array scanners, fluorometers and flow cytometers. Flow cytometers are particularly well suited for measuring the detectable signal within cells directly In alcohol solutions containing PNA probes. Advantages to flow cytometry systems include easy customization to field applications or bedside (point of care) testing, rapid results (less than about 3 hours), and minimal manual steps. A flow cytometer adapted for point of care testing by addition of thermal regulation (potentially just a heat block) would be a particularly useful system for the measurement of detectable signal within cells directly in alcohol solutions containing PNA probes.

Further advantages provided by the invention include simplified sample preparation and use. For instance, and for many embodiments, sample preparation (eg., fixation) and analysis (hybridization) may be performed in one or just a few steps using a single solution. According to this illustration of the invention, the number of sample preparation steps can be significantly reduced or eliminated entirely. Such sample preparation steps are often complicated and time consuming processes that are generally performed prior to probe based assays. It should be noted that assays described in the literature as "homogeneous", such as real-time PCR, typically involve several labor intensive steps to prepare the sample prior to addition of the sample into the assay. Thus, practice of the invention simplifies prior analysis of target nucleic acid samples.

As discussed, the Invention is well suited for methods of analysis of at least one target nucleic acid in a sample. Thus in one embodiment, the method includes
a) isolating and optionally amplifying nucleic acid; b) contacting the isolated and optionally amplified nucleic acid with at least one PNA probe in an aqueous alcohol solution comprising from 40% (v/v) to 60% (v/v) alcohol, wherein the probe specifically binds any target nucleic acid therein; and c) detecting the bound PNA probe as being indicative of the presence of the target nucleic acid in the sample.

The invention also includes a kit adapted to analyze at least one target nucleic acid in a sample of interest. In one invention embodiment, the kit includes at least one PNA probe that specifically binds the target nucleic acid; provided in an aqueous alcohol solution comprising from 40% (v/v) to 60% (v/v) of at least one lower alcohol; and directions for using the kit. Thus, the kit may be adapted to samples that are already in an aqueous alcohol solution or samples that are treated with the aqueous alcohol solution as part of the kit procedure.

In related embodiments, the invention provides methods and compositions useful for detecting one or more target substances in an alcoholic preservative solution. Additionally, the invention can be used to Identify what is sometimes referred to as "sensors" that can be used to bind such targets. Unless otherwise specified, preferred sensors will include or consist of PNA. Preferred methods allow for the simultaneous performance of sufficient fixation of a sample and binding of a detectable sensor to a target of interest in the sample. In one aspect, a method is provided that comprises contacting a sample suspected of containing a target with a detectable sensor molecule (eg., a PNA probe as described herein) known to bind to such target In an alcoholic preservative solution. The method may be performed in multiplex form to permit simultaneous analysis of a plurality of targets. Methods for identifying a sensor capable of binding to a desired target in an alcoholic preservative solution are also provided. An alcoholic preservative solution comprising one or more such detectable sensors is also provided. Also provided is a sample comprising a bound sensor provided by such a process. Also provided are kits useful for such methods. Sensors can be used in any of the methods described herein and may be labeled or unlabeled according to the present disclosure depending on intended use.

Other uses, advantages and embodiments of the invention are described below.

### Detailed Description of the Invention

As discussed, the invention provides methods and kits for hybridizing at least one PNA probe (eg., less than about four, preferably one or two of such probes) to a target with an aqueous alcohol solution. A preferred solution includes one or a combination of alcohols (preferably one or two) that can be used, for instance, as a specimen fixing and probe hybridization medium. The invention has a wide spectrum of Important applications including use in the detection of pathogens (eg., microbes and viruses) in a sample having clinical, forensic, public safety, veterinary, environmental or medical interest.

The invention provides a wide variety of compositions to suit an intended use. In one embodiment, the aqueous alcohol solution includes sufficient lower alcohol to fix cells within a few minutes or less following contact with the composition. By the phrase "aqueous alcohol solution" is meant a water based solution that includes a lower alcohol which is miscible in water. The solution includes between from about 40 to about 60% (v/v) of the lower alcohol. By "lower alcohol" is meant a water miscible alcohol having three or less carbon atoms ie., ethanol, methanol, and isopropanol (2-propanol). An invention composition can include one or more than one lower alcohol as required (eg., one, two, or three).

An aqueous alcohol solution In accord with the invention can further include at least one of the following components: a buffering agent (eg., Tris, Hepes, PBS, etc); monovalent cation such as sodium or potassium; divalent cation such as calcium or magnesium ion; suitable anion such as acetate, chloride, fumarate, maleate, citrate, sulfate, phosphate, carbonate, etc.; cross-linking agent such as formaldehyde; chaotropic agent such as formamide; chelating agent such as EDTA; ionic or non-ionic detergent such as Triton-X-100, Nonidet P-40, SDS; stabilizer such as a polymer (eg., Ficoll 400, dextran salt, etc); or a preservative (eg., azide). In certain invention embodiments, one or more of the components may be present in trace amounts. For other applications, specific amounts of the components to be added to the solution will be informed by recognized parameters such as the sample for which detection of the target is needed, the assay format to be used, etc.

Additionally preferred aqueous alcohol solutions provide a suitable hybridization medium that supports specific binding between the PNA probe and the target nucleic acid. By "specific binding" or similar term is meant that the PNA probe and target nucleic acid form a complex (specific binding pair) in the solution that is readily detectable by standard methods eg., Southern blot hybridization, fluorescence in situ hybridization and related assays. Typically, but not exclusively, the PNA probe does not recognize or bind other target nucleic acids. See the following references for information relating to detecting specific binding between molecules. Sambrook et al. in Molecular Cloning: A Laboratory Manual (2d ed. 1989); and Ausubel et al., Current Protocols in Molecular Biology, John Wiley & Sons, New York, 1989;

In one embodiment, the aqueous alcohol solution includes at least one and preferably all of the following components: between about 45 to 55% (v/v) methanol, between about 2 to 4% EDTA (w/v), and between about 6 to 8% (w/v) sodium acetate.

In embodiments in which the addition of formamide to the aqueous alcohol solution is desired, that solution will include between about 0 to 70% (v/v) formamide.

Preferred use of the invention provides other benefits and advantages. For Instance, It is an object of the invention to provide compositions in which the aqueous alcohol solution component serves as a fixation and/or hybridization solution. Such use is intended to help minimize or in some embodiments eliminate the use of hazardous or toxic chemicals. Examples of such chemicals are 30% formamide (Williams et al. Methods Mol Biol. 208:181-93 (2002), 4% gluteraldehyde (Perry-O'Keefe et al., J. Microbiol. Methods 47:281-292 (2001)), and other cross-linking reagents typically used for fixation of cells for optimal performance of fluorescence in situ hybridization assays using either DNA or PNA probes.

### I. Definitions:

a. As used herein, the term "nucleobase" means those naturally occurring and those non-naturally occurring heterocyclic moieties commonly known to those who utilize conventional nucleic acid, locked nucleic acid or peptide nucleic acid technology to thereby generate polymers that can sequence specifically bind to nucleic acids.
b. As used herein, the term "nucleobase sequence" means any segment of a polymer that comprises nucleobase-containing subunits. Non-limiting examples of suitable polymers or polymer segments include oligodeoxynucleotides, oligoribonucleotides, locked nucleic acids, peptide nucleic acids, nucleic acid analogs, nucleic acid mimics, and/or chimeras.
c. As used herein, the term "target sequence" means the nucleobase sequence of the target nucleic acid that is to be detected in an assay. Detection of a target sequence is frequently indicative of the presence of a particular organism, disease state, or genetic type.
d. As used herein, the term "probe" means a polymer (e. g. a DNA, RNA, PNA, chimera or linked polymer) having a probing nucleobase sequence that is designed to sequence-specifically hybridize to a target sequence of interest.
e. As used herein, "analyze" means detection, identification, typing and/or quantitation a target nucleic acid.
f. As used herein, the term "peptide nucleic acid" or "PNA" means any oligomer, linked polymer or chimeric oligomer, comprising two or more PNA subunits (residues), including any of the polymers referred to or claimed as peptide nucleic acids in United States Patent Nos. 5,539,082, 5,527,675, 5,623,049, 5,714,331, 5,736,336, 5,773,571, 5,786,461, 5,837,459, 5,891,625, 5,972,610, 5,986,053, 6,107,470 and 6,357,163. In the most preferred embodiment, a PNA subunit consists of a naturally occurring or non-naturally occurring nucleobase attached to the aza nitrogen of the N- [2-(aminoethyl)] glycine backbone through a methylene carbonyl linkage.
g. As used herein, the terms "label" and "detectable moiety" are interchangeable and shall refer to moieties that can be attached to a probe to thereby render the probe detectable by an instrument or method.

### 2. Description

### I. General:

### PNA Synthesis:

Methods for the chemical assembly of PNAs are well known (see: US Patent Nos. 5,539,082, 5,527,675, 5,623,049, 5,714,331, 5,736,336, 5,773,571, 5,786,461, 5,837,459, 5,891,625, 5,972,610,-5,986,053 and 6,107,470).

### PNA Labeling:

Preferred non-limiting methods for labeling PNAs are described in US 6,110,676, 6,361,942, 6,355,421 or are otherwise well known in the art of PNA synthesis and peptide synthesis.

### Labels:

Non-limiting examples of detectable moieties (labels) suitable for labeling high affinity probes used in the practice of this invention would include a dextran conjugate, a branched nucleic acid detection system, a chromophore, a fluorophore, a lanthanide a spin label, a radioisotope, an enzyme, a hapten, an acridinium ester and a chemiluminescent compound.

Other suitable labeling reagents and preferred methods of attachment would be recognized by those of ordinary skill in the art of PNA, peptide, or nucleic acid synthesis.

Preferred haptens include 5 (6)-carboxyfluorescein, 2,4-dinitrophenyl, digoxigenin, and biotin.

Preferred fluorochromes (fluorophores) include 5 (6)-carboxyfluorescein (Flu), tetramethyl-6-carboxyrhodamine (tamra), 6- ((7- amino-4-methylcoumarin-3-acetyl) amino) hexanoic acid (Cou), 5 (and 6)-carboxy-X- rhodamine (Rox), Cyanine 2 (Cy2) Dye, Cyanine 3 (Cy3) Dye, Cyanine 3.5 (Cy3.5) Dye, Cyanine 5 (Cy5) Dye, Cyanine 5.5 (Cy5.5) Dye Cyanine 7 (Cy7) Dye, Cyanine 9 (Cy9) Dye (Cyanine dyes 2,3,3.5,5 and 5.5 are available as NHS esters from Amersham, Arlington Heights, IL), JOE, Tamara or the Alexa dye series (Molecular Probes, Eugene, OR).

Preferred enzymes include polymerases (e. g. Taq polymerase, Klenow PNA polymerase, T7 DNA polymerase, Sequenase, DNA polymerase 1 and phi29 polymerase), alkaline phosphatase (AP), horseradish peroxidase (HRP) and most preferably, soy bean peroxidase (SBP).

### Self-Indicating Probes:

Beacon probes are examples of self-indicating probes which include a donor moiety and a acceptor moiety. The donor and acceptor moieties operate such that the acceptor moieties accept energy transferred from the donor moieties or otherwise quenches signal from the donor moiety. Though the previously listed fluorophores (with suitable spectral properties) might also operate as energy transfer acceptors, preferably, the acceptor moiety is a quencher moiety. Preferably, the quencher moiety is a non-fluorescent aromatic or heteroaromatic moiety. The preferred quencher moiety is 4-((-4-(dimethylamino) phenyl) azo) benzoic acid (dabcyl). In a preferred embodiment, the self-indicating Beacon probe is a PNA Linear Beacon as more fully described in US 6,485,901.

In another embodiment, the self-indicating PNA probes of this invention are of the type described in WIPO patent application W097/45539. These self-indicating probes differ as compared with Beacon probes primarily in that the reporter must interact with the nucleic acid to produce signal.

### Spacer/Linker Moieties:

Generally, spacers are used to minimize the adverse effects that bulky labeling reagents might have on hybridization properties of probes. Preferred spacer/linker moieties for the nucleobase polymers of this invention consist of one or more aminoalkyl carboxylic acids (e. g. aminocaproic acid), the side chain of an amino acid (e. g. the side chain of lysine or omithine), natural amino acids (e. g. glycine), aminooxyalkylacids (e. g. 8-amino-3,6-dioxaoctanoic acid), alkyl diacids (e. g. succinic acid), alkyloxy diacids (e. g. diglycolic acid) or alkyldiamines (e. g. 1, 8-diamino-3, 6-dioxaoctane).

### Fixation

Fixation means specimen preservation and/or sterilization where cellular nucleic acid (DNA and RNA) integrity and cellular morphology are essentially maintained. Fixation is typically performed either chemically using one or more solutions containing one or more fixing agent(s) and/or mechanically, such as for example by preparation of a smear on a microscope and subsequently heating the smear either by passing the slide-through a flame or placing the slide on a heat block. Fixation is also used to make the cell wall permeable to probes for in situ hybridization assays.

### Fixation Solution

Fixation Solution means solutions used for chemical fixation of specimens.

### Suitable Hybridization Conditions:

Generally, the more closely related the background causing nucleic acid contaminants are to the target sequence, the more carefully stringency must be controlled. Blocking probes may also be used as a means to improve discrimination beyond the limits possible by optimization of stringency factors. Suitable hybridization conditions will thus comprise conditions under which the desired degree of discrimination is achieved such that an assay generates an accurate (within the tolerance desired for the assay) and reproducible result.

Aided by no more than routine experimentation and the disclosure provided herein, those of skill in the art will easily be able to determine suitable hybridization conditions for performing assays utilizing the methods and compositions described herein. Suitable in-situ hybridization comprises conditions suitable for performing in-situ hybridization procedures. Thus, suitable in-situ hybridization conditions will become apparent to those of skill in the art using the disclosure provided herein, with or without additional routine experimentation.

### Blocking Probes:

Blocking probes are nucleic acid or non-nucleic acid probes that can be used to suppress the binding of the probing nucleobase sequence of the probing polymer to a non-target sequence. Preferred blocking probes are PNA probes (see: US 6,110, 676). It is believed that blocking probes operate by hybridization to the non-target sequence to thereby form a more thermodynamically stable complex than is formed by hybridization between the probing nucleobase sequence and the non-target sequence. Formation of the more stable and preferred complex blocks formation of the less stable non-preferred complex between the probing nucleobase sequence and the non-target sequence. Thus, blocking probes can be used with the methods, kits and compositions of this invention to suppress the binding of probes to a non-target sequence that might be present and interfere with the performance of the assay.

### Probing Nucleobase Sequence:

The probing nucleobase sequence of a probe of this invention is the specific sequence recognition portion of the construct. Therefore, the probing nucleobase sequence is a nucleobase sequence designed to hybridize to a specific target sequence of interest in a sample. Consequently, with due consideration to the requirements of a probe for the assay format chosen, the length and sequence composition of the probing nucleobase sequence of the probe will generally be chosen such that a stable complex is formed with the target sequence under suitable hybridization conditions.

### II. Preferred Embodiments of the Invention:

### a. PNA Probes:

As discussed, in one aspect the invention relates to one or more PNA probe(s) in an aqueous alcohol solution where said PNA probe(s) includes a nucleobase sequence complementary or substantially complementary to the target sequence of interest. Preferred PNA probes will have a length that is generally between 8 and 35 subunits, preferably less than about 20 subunits with between from about 12 to about 18 subunits being preferred for many applications.

A probe of this invention will generally have a probing nucleobase sequence that is exactly complementary to the target sequence. Alternatively, a substantially complementary probing nucleobase sequence might be used since it has been demonstrated that greater sequence discrimination can be obtained when utilizing probes wherein there exists one or more point mutations (base mismatch) between the probe and the target sequence (See: Guo et al., Nature Biotechnology 15: 331-335 (1997)). Consequently, the probing nucleobase sequence may be only about 90% homologous to the probing nucleobase sequences identified above. Substantially complementary probing nucleobase sequence within the parameters described above is considered to be an embodiment of this invention.

Further probes may preferably be labeled with a detectable moiety, such as but not limiting to a conjugate, a branched detection system, a chromophore, a fluorophore, a lanthanide, a spin label, a radioisotope, an enzyme, a hapten, an acridinium ester and a luminescent compound. Examples of labels have been described above.

Further probes in accord with the invention are self-indicating (or self-reporting) probes which preferably have a PNA Linear Beacon format as described herein.

Further probes according to the invention will include at least one spacer or linker group that is preferably adapted to help space the detectable moiety from the probing sequence. A variety of suitable spacer/linkers have already been described.

Further the PNA probes may be independently detectable probes for independent analysis of two or more target sequences of interest, ie., multiplex analysis

It may be preferable to use one or more unlabeled probes in the assay to thereby suppress the binding of the detectable PNA probe(s) to a non-target sequence. The presence of the "blocking probe(s)" helps to increase the discrimination of the assay and thereby improve reliability and sensitivity (signal to noise ratio).

The PNA probes may be designed to specifically bind nucleic acid targets, such as both DNA and RNA, including rRNA and mRNA. Non-limiting examples, includes PNA probes for analysis of microorganisms and for example targeting species-specific rRNA sequences. For example the PNA probes may be targeting microorganisms potentially present in cervical swabs associated with women's health, such as but not limited to PNA probes targeting Chlamydia trachomatis and/or Neisseria gonorrhoeae (for examples of PNA probe nucleobase sequences see US 6169169), Candida albicans (for example of PNA probe nucleobase sequences see Rigby et al. J. Clin. Microbiol. 40:2182-218(2002)), Gardnerella vaginalis, Trichomonas vaginalis and/or Streptococcus agalactia (Group B streptococci).

PNA probes targeting DNA or mRNA of one or more genes of interest are also within the embodiment of this invention. Non-limiting examples include oncogenes and viruses, for example human papillomavirus.

Particular amounts of PNA probe to use with the invention will vary according to recognized parameters such as the level of assay sensitivity and/or selectivity needed, the particular probe and label used, sample amount, etc. However, for most applications it will be useful to have between about 0.1 nM to 1500nM of the probe, preferably about 10 to about 500 nM of the probe. In many embodiments, compositions according to the invention will be combined with the aqueous alcohol solution and include the specified amounts of PNA probe.

In embodiments in which inclusion of a control sample is desired, that sample may include all constituents of an actual (experimental) sample except one or more of the PNA probe and target nucleic acid (cells). In embodiments in which it is useful to provide a known quantity of target nucleic acid (eg., when a detection device needs to be calibrated), an acceptable amount will be about 500nM. Of course, for many invention applications in which the characteristics of a particular sample are known, for instance, as in many clinical specimens, it will not always be necessary to employ a control sample to optimize use of the invention.

### b. Aqueous Alcohol Solutions

Aqueous alcohol solutions are commonly used as part of the sample preparation process prior to performing probe-based assays, however the use of alcohols are always combined with other mechanical or chemical agents. Aqueous alcohol solutions are not recommended for the hybridization step due to the denaturing and precipitating effect of alcohol on nucleic acid probes. In accordance with this invention PNA probes perform very well in alcohol solutions such that alcohol solutions can be used for both sample preparation and/or subsequent analysis using PNA probes and thus enable development of both traditional as well as novel assay formats that are impossible using DNA probes and therefore go above and beyond what has previously been possible within the area of probe-based analysis.

In one embodiment, the alcohol solutions of this invention comprise between 40% and 60% (v/v).

In many embodiments, the aqueous alcohol solution is used in accord as a fixing agent. It therefore may contain additional chemicals for optimal fixation, such as an aqueous solution comprising a water-miscible alcohol and a buffering agent which maintains said solution with said cells at a pH range of between about 2 and about 7. in one preferred embodiment, the solution comprises methanol, EDTA, and sodium acetate. In that embodiment, the solution constitutes about 45-55% (w/v) methanol, 2-4 % (w/v) EDTA and the sodium acetate buffer constitutes about 6-8% (w/v).

As discussed, the aqueous alcohol solution may also contain a chaotrophic chemical, such as formamide in a concentration of 10-70% (v/v).

In accordance with the invention the alcohol solution may be a fixing agent for biological samples, such as but not limited to cervical swabs. Most preferably, the alcoholic solution is PreservCyt™ Reagent (Cytyc Corp, Boxborough, MA) or SurePath™ Preservation Fluid (TriPath Imaging, Inc., Burlington, NC) or equivalent solutions are non-limiting examples of commercially available alcohol-based fixation solutions for clinical specimens.

### c. Methods:

Use of the invention Is also flexible in the sense that It can be used in a wide variety of assay formats, including formats which go beyond what is possible with traditional DNA probes and conventional hybridization solutions.

As discussed, the invention provides methods for the analysis of a target nucleic acid In a sample of Interest. In one embodiment, the sample includes cells and the method includes at least one of and preferably all of the following steps: a) contacting the cells with at least one PNA probe in an aqueous alcohol solution comprising from 40% (v/v) to 60% (v/v) alcohol, wherein the probe specifically binds any target nucleic acid; b) incubating the cells and PNA probe(s); and c) detecting any bound PNA probe as being indicative of the presence of the target nucleic acid in the cells.

The method is flexible and can be adapted as needed to suit an intended invention use. For instance, cells may be fixed at nearly any suitable step including before, during or after step (a). In one embodiment, the method includes fixing the cells prior to step (a) of the method. Typically, use of an aqueous alcohol solution according to the invention will be preferred. However, in certain other embodiments other fixing agents may be used such as those disclosed herein.

In another embodiment of the method, the cells are fixed during step (a) and (b), preferably with the aqueous alcohol solution

According to step (b) of the method, the cells may be incubated under nearly any condition conducive to allowing penetration of the PNA probe into the cells. For instance, the incubation step can be performed for at least 10 minutes (eg., between about 10 minutes and 24 hours or even longer) at between about 18 to 60 °C. Particular reaction conditions to use will be guided by recognized parameters such as the intended application, the specific PNA probe used, the level of assay sensitivity needed, etc.

Step (c) of the method can be conducted using nearly any suitable detection format such as by microscopy, flow cytometry and related methods. In embodiments in which visual inspection of the cells is advisable, the method can further include the step of placing the cells onto a microscope slide. Preferably, the placing the cells onto a microscope slide is performed prior to step (c) of the method.

For some invention applications it will be helpful to filter the cells manipulated in the method. Such a step can be performed at nearly any point in the method including before, during or after steps (a)-(c). In one embodiment, the filtered cells are placed on the microscope slides prior to step (b).

Optionally, and in one embodiment, the invention method can further include the step of removing any unbound PNA probe prior to the detection step (c).

Other embodiments of the method are contemplated. For instance, the cells can be fixed prior to performing the method using standard fixation procedures which may or may not involve aqueous alcohol solutions, exemplified in Example 3-7. In a preferred embodiment, the cells are fixed using the same aqueous alcohol solution as used in step (a) as exemplified in Example 7 and in a more preferred embodiment, the cells are fixed during step (a) using the aqueous alcohol solution and in the presence of PNA probes as exemplified in Example 4.

Binding of the PNA probe to the target nucleic acid may be performed under suitable hybridization conditions which typically involve incubation for certain time periods and at certain temperatures as exemplified in the various examples below. Typically, the hybridization reaction takes place at ambient temperature or above for at least 10 minutes.

Prior to detecting the bound probe in step (c) the sample may or may not be transferred. For example the sample may be transferred onto a microscope slide either directly or following a filtration or centrifugation step or analyzed by flow cytometry.

The invention is particularly well suited for use in conjunction with morphological staining procedures of cells or tissue, such as for example PAP staining where analysis using PNA probes is performed before, during or after step (a) of the method.

As apparent from the description above and the examples below this invention is particularly well suited for in situ hybridization, including fluorescence in situ hybridization, but other assay formats benefiting from the advantages of using PNA probes in alcohol solutions are also within the scope of this invention.

In accordance with this invention for analysis of a target nucleic acid in a sample, the method comprises a) isolating and optionally amplifying nucleic acid (eg., by PCR or related method), b) contacting the isolated and optionally amplified nucleic acid with at least one PNA, probe in an aqueous alcohol solution comprising from 40% (v/v) to 60% (v/v) alcohol, wherein the probe specifically binds any target nucleic acid therein; and c) detecting the bound PNA probe as being indicative of the presence of the target nucleic acid in the sample.

As discussed, the invention also features a method for analysis of a target nucleic acid in a sample. In one embodiment, the method includes at least one of and preferably all of the following steps: a) isolating and optionally amplifying nucleic acid, b) contacting the isolated and optionally amplified nucleic acid with at least one PNA probe in an aqueous alcohol solution comprising from 40% (v/v) to 60% (v/v) alcohol, wherein the probe specifically binds any target nucleic acid therein; and c) detecting the bound PNA probe as being indicative of the presence of the target nucleic acid in the sample. More specific contact, incubation, and detection steps have already been discussed and can be used, as needed, to practice this invention method.

As also discussed, the invention features a method for detecting a human papillomavirus in cervical samples. In one embodiment, the method includes at least one of, and preferably all of the following steps: a) obtaining a cervival sample from a patient, b) contacting the sample with aqueous alcohol solution comprising from 40% (v/v) to 60% (v/v) alcohol containing at least one PNA probe (eg., less than about four, preferably one or two) that specifically binds DNA or mRNA of human papillomavirus, c) incubating the sample with the aqueous alcohol solution; and d) detecting any bound PNA probe as being indicative of the presence of the human papillomavirus in the sample.

In this embodiment, the clinical analysis of patient cervical samples is intended. Appropriate methods for obtaining the cervical sample are known In the field. More specific contact, incubation and detection steps have already been discussed and can be used, as needed, to practice this invention method. A particular invention composition includes at least one PNA probe that specifically binds human papillomavirus DNA or RNA encoded by same in combination with at least one (eg., one, two or three) aqueous alcohol solution.

As also mentioned, the invention features a kit adapted to detect a target nucleic acid in a sample of interest. In one embodiment, the kit includes all of the following components: a) at least one PNA probe (eg., one, two, three or four) that specifically binds the target nucleic acid provided in (b) an aqueous alcohol solution comprising from 40% (v/v) to 60% (v/v) of at least one lower alcohol; and c) directions for using the kit. Particular kits of the invention feature the above-mentioned components (a) and (b) combined in a single vessel . It is contemplated that some kits may include a variety of PNA probes, labels, and/or aqueous alcohol solutions to help optimize use of the invention.

### Exemplary In Situ Hybridization Assay Formats:

Exemplary methods for performing PNA-based in situ hybridization assays can be found in: Oliveira et., J. Clin. Microbiol 40:247-251 (2002), Rigby et al., J. Clin. Microbiol. 40:2182-2186 (2002), Stender et al., J. Clin. Microbiol. 37:2760-2765 (1999), Perry-O'Keefe et al., J. Microbiol. Methods 47:281-292 (2001). Modifications of the procedures where one or more manual steps, such as centrifugation or wash steps, are eliminated (XI et al., Appl. Environ. Microbiol 69:5673-5678 (2003)) and novel assay formats such as those exemplified below are also within the scope of this invention.

Measurement of the detectable signal within the cells, may be performed by a variety of instruments, such as but not limited to the following examples: bright field microscope (for example see Thisted) fluorescence microscope (for example see Oliveira et al., J. Clin. Microbiol 40:247-251 (2002)), film (for example see Perry-O'Keefe et al., J. Appl. Microbiol. 90:180-189) (2001), camera and instant film (for example see Stender et al., J. Microbiol. Methods 42:245-253 (2000)), luminometer (for example see Stender et al., J. Microbiol. Methods. 46:69-75 (2001), laser scanning device (for example see Stender et al., J. Microbiol. Methods. 45: 31-39 (2001) or flow cytometer (for example see Wordon et al., Appl. Environ. Microbiol. 66:264-289 (2000)). Automated slide scanners and flow cytometers are particularly useful for rapid and/or quantitative measurements.

### Other Exemplary Assay Formats:

The invention is also well suited for other assay formats. In particular, the use of PNA probes in alcohol solutions enables development of very simple assay formats, including dosed tube assays or 1-step assays (a.k.a. "homogeneous assays"). By closed tube we mean that once the components of the assay have been combined, there is no need to open the tube or remove, replace or add components in order to complete the assay. In closed tube assays the tube need not, and preferably will not, be opened to determine the result. There must therefore be some detectable or measurable change which occurs and which can be observed or quantitated without opening the tube or removing the contents of the assay. Thus, most dosed tube assays rely on a change in fluorescence which can be observed with the eye or can be detected otherwise and/or quantitated with a fluorescence instrument which uses the tube as the sample holder, in contrast 1-step assays require removal of the content prior to determining the results, e.q. placing the content on a microscope slide for analysis by microscopy or analysis of the content by flow cytometry.

### d. Kits:

In yet another embodiment, this invention is directed to kits suitable for performing a hybridization assay using PNA probes in an aqueous alcohol solution. The general and preferred characteristics of PNA probes and aqueous alcohol solution have been previously described herein. Furthermore, methods suitable for using PNA probe(s) in alcohol solutions have been previously described herein.

In one kit embodiment, it will often be helpful to include a positive control, although it will be appreciated that use of the positive control need not accompany every invention application such as when the properties of a particular sample or sample set are well known (eg., clinical samples).

### e. Exemplary Applications For Using The Invention:

The methods, kits and compositions of this invention are particularly useful for the analysis of clinical samples, e.g. urine, blood, wounds, sputum, cervical swabs, gastric lavage, bronchial washings, biopsies, aspirates, expectorates as well as in food, beverages, water, pharmaceutical products, personal care products, dairy products or environmental samples and cultures thereof. For example, the assay may be used to detect a target sequence which is specific for a genetically based disease or is specific for a predisposition to a genetically based disease. Non-limiting examples of diseases include beta-Thalassemia, sickle cell anemia, Factor-V Leiden, cystic fibrosis and cancer related targets such as p53, p10, BRC-1 and BRC-2. The assay may also be used to detect, identify and/or quantify microorganisms optionally present in the sample, such as but not limited to pathogens in cervical swabs, such as but not limited to Chlamydia trachomatis and human papilomavirus or bioterrorism agents, such as but not limited to Bacillus anthracis, In environmental samples

In still another embodiment, the target sequence may be related to a chromosomal DNA, wherein the detection, identification or quantitation of the target sequence can be used in relation to forensic techniques such as prenatal screening, paternity testing, identity confirmation or crime investigation.

The present invention can be used with at least certain compositions and methods provided by the following references: WO2005/007872 (PCT/US2004/022390) as filed on July 11, 2003; and US patent publication 2004/0115692 as filed on July 11, 2003.

Having described the preferred embodiments of the invention, it will now become apparent to one of skill in the art that other embodiments incorporating the concepts described herein may be used.

### EXAMPLES

### Example 1 - Fluorescent Tm in an aqueous alcohol Solution

DNAs were designed to represent the regions of the 16S ribosomal genes of Staphylococcus aureus (Sa16), and Staphylococcus schleiferi (Ss16), which differ by only a few bases. S. aureus and S. schleiferi are very closely related phylogentically, and are thus difficult to discriminate by conventional assays.
The sequences of the DNA targets are, as follows.
Sa16: AGC [GAACGGACGAGAAGC] TTG CTT CTC TGA TGT TAG
Ss16: AGC [GAACGGACGAGGAGC] TTG CTC CTT TGA AGT TAG

Note that the regions complementary to the PNA probe Sta03-Flu are contained within brackets, and the divergent bases between the two target sequences are underlined. DNA targets were obtained from Applied Biosystems. The PNA probe, Sta-LS (also obtained from Applied Biosystems) has the following sequence and labeling, where Flu indicates the fluorescent label 6-fluorescein, O represents the linker group 8-amino-3,6-dioxaoctanoic acid, Lys is the amino acid lysine, and dabcyl is the quencher group 4-((-4-(dimethylamino) phenyl) azo) benzoic acid.

Sta-LS: Flu-OO-GCT TCT CGT CCG TTC-Lys-Lys(Dabcyl) (Probe sequence obtained from Perry-O'Keefe et al., J. Appl. Microbiol. 90:180-189 (2001)).

Samples were prepared by diluting 2.5 uL of the Sta-LS probe (50 uM stock) into 492.5 uL of either TE (10 mM Tris-Cl, 1 mM EDTA pH 9.0), or PreservCyt (pH adjusted with NaOH, see below) and 5 uL of DNA Sa16, or Ss16 (both at 50 uM), or 5 uL water for the no target controls. Final concentrations were 250 nM probe and 500 nM targets.

PreservCyt reagent (Cytyc Corp.) was adjust to pH - 9.0 from the stock pH of ∼5 (values obtained by pH paper) by adding 12 uL of 2N NaOH to 4 mL PreservCyt. The pH of the PreservCyt solution was adjusted to allow comparison to the TE reagent (also at pH 9.0), and because fluorescein's fluorescence is dramatically reduced at low pH.

Samples were prepared as described above and placed into an optically clear 96 well plate. For each target, and a no target control, triplicate samples were made of 25 uL each. The plate was loaded into the BioRad iCycler instrument, a thermocycler with an attached fluorometer. Samples were first heated to 80°C for 1 minute, then cooled to 30°C. Next the samples were heated at a rate of 0.5°C per minute, from 30°C to 80°C while fluorescent measurements were made for each sample, at each 0.5°C interval.

The instrument software plots temperature vs. fluorescence for each sample, and automatically performs a 1^{st} derivative analysis of each data set to determine the melting temperature of the sample. Samples in which hybrids are melted display a characteristic sigmoidal curve. The 1^{st} derivative analysis determines the inflection point in the curve, which is defined as the melting temperature of a hybrid (the point at which the limiting strand of a hybrid is 50% free in solution, and 50% hybridized).

Of the samples tested, both of the no target controls have temperature vs, fluorescence data which displayed a slow but steady increase in signal as the temperature increases. The no target controls had average signal increases from 30°C to 80°C of approximately 47% for the PreservCyt samples, and about 37% for the TE samples. This pattern is characteristic of PNA LightSpeed probes which become more randomly oriented as temperatures increase, and as a result display less fluorescent quenching than at cooler temperatures.

The samples which contained either the Sa16 or Ss16 DNA targets all displayed the characteristic sigmoidal curves associated with the melting of PNA Linear Beacon probe hybrids. Samples in PreservCyt had and average decrease in fluorescent signal (from 30°C to 80°C) of -72%, and those in TE an average decrease of -80%. These decreases in signal combined with the sigmoidal shaped curves are highly indicative of PNA Linear Beacon probe hybrid melts. The corresponding 1^{st} derivative curves all display sharp peaks at the inflection points of the PNA melts, indicating the Tms of these hybrids. The average hybrid Tms are listed below in Table1.

**Table 1**

| | 1 | 2 | 3 | 4 | 5 | 6 |
|---|---|---|---|---|---|---|
| **A** | **Reagent** | **Target** | **RLU 30°C** | **RLU 80°C** | **%Change 30°C-80°C** | **Tm** |
| B | PreservCyt | none | 313 | 461 | 47% | No Tm |
| C | PreservCyt | Sa16 | 1450 | 391 | -73% | 60.7 |
| D | PreservCyt | Ss16 | 850 | 248 | -71% | 53.7 |
| E | TE | none | 72 | 98 | 36% | No Tm |
| F | TE | Sa16 | 424 | 80 | -81% | 66.3 |
| G | TE | Ss16 | 364 | 78 | -79% | 56.8 |

With reference to Table (1), in both reagents examined, the Tm differences observed between the Sa16 and Ss16 targets are indicative of the hybrid destabilization typically observed for a single base mismatch. The values in columns 3 and 4 of Table 1 are in relative light units (RLU), and represent the brightness of fluorescence in each sample at the given temperatures. The percent change in Tm between the match and the mismatch targets ((match-mismatch)/match) are -11.5% and -14.3% for PreservCyt and TE respectively. These data indicate that both hybridization and the self-reporting capabilities of PNA Linear Beacons occur in the alcohol based commercial preservative, PreservCyt, in a similar manner as in TE.

### Example 2 - Slide-based hybridization in aqueous alcohol solutions

An experiment was performed to examine the possibility of using alcohol solutions as hybridization reagents in slide based fluorescent in situ hybridization (FISH) assays. A comparison was made between two commonly encountered Staphylococcus species which are frequently found in positive blood culture bottles. *Staphylococcus aureus* is a human pathogen commonly found in nosocomal infections, and *Stapylococcus epidermidis* is a frequent contaminant of blood cultures. Slides were prepared with either *Staphylococcus aureus* (ATCC# 29213) or *Staphylococcus epidermidis* (ATCC# 14990) pure cultures using conventional methods. Briefly, the bacteria were grown overnight in an appropriate nutrient medium, a drop of culture and a drop of fixation solution (AdvanDx, Woburn, MA) were mixed together on a ThinPrep microscope slide (Cytyc, Boxborough, MA MA), and the slides were placed on a 60°C heat block for 20 minutes. The heat step dries the sample onto the slide and fixes the bacterial cells. Slides were stored at room temperature until they were used.

Labeled PNA probes were obtained from Applied Biosystems (Foster City, CA), a Flu labeled 15-mer which is complementary to the *Staphylococcus aureus* rRNA sequence (Sta03-Flu), and a TAM labeled 15-mer complementary to the *Staphylococcus epidermidis* rRNA sequence (Sep01-TAM). The rRNA sequences of the two Staphylococcus species are very closely related, but have significant differences such that the two probes are sequence specific. When aligned to the *Staphylococcus epidermidis* rRNA sequence, the Sta03 probe has mismatches in two positions. When aligned to the *Staphylococcus aureus* rRNA sequence, the Sep01 probe has one mismatch, and one gap.
Sta03-FLU: Flu-OO-GCT TACT CGT CCG TTC (Perry-O'Keefe et al., J. Appl. Microbiol. 90:180-189) (2001)
Sep01-TAM: TAMRA-OO-GCT CCA AAT GGT TAC (Own patent application)

Fluorescein (Flu) and tetramethyl-6-carboxyrhodamine (TAM) are both fluorescent labels which glow green and red respectively when properly excited. Six different hybridization buffer formulations were prepared, each containing either 500 nM Sta03-Flu, or 500 nM of both Sta03-Flu and Sep01- - TAM. Hybridization reagent B, the control formulation commonly used in FISH assays using PNA probes, is 30% formamide, 10% dextran sulfate, 1% Triton-X 100, 0.2% polyvinylpyrillidone, 0.2% ficoll 400, 0.1% sodium pyrophosphate, 5 mM ethylenediaminetetracetic acid, 10 mM sodium chloride, 50 mM Tris pH 7.6. Hybridization reagent C is PreservCyt Reagent (Cytyc Corp). Hybridization reagent D is SurePath Preservative Fluid (TriPath Imaging, Inc, Burlington, NC). Hybridization reagent E is 50% methanol in water. Hybridization reagent F is 50% ethanol in water. Hybridization reagent G is 50% reagent alcohol (VWR # VW3609-1, 90.25% ethanol, 4.75% methanol, 5% isopropanol) in water.

Hybridizations were performed by adding 50 uL of a hybridization formulation to a slide, covering the slide with a cover glass, and incubating the slide on a heater block at 50 °C for 30 minutes. Cover glasses were removed, and all slides were washed together at 60 °C for 30 minutes in a staining dish containing 200 mL of 5 mM Tris, pH 10, 15 mM sodium chloride, 0.1% Triton X-100. After air drying, one drop of Mounting Medium (AdvanDx) and a cover glass were added to each slide. Slides were visualized on an Olympus BX51 fluorescent microscope equipped with a 60X oil immersion objective and a FITC/Texas Red dual band filter for simultaneous observation of fluorescence from Flu and TAM.

The results are displayed in Table (2).

**Table (2)**

| | 1 | 2 | 3 | 4 | 5 |
|---|---|---|---|---|---|
| A | **Reagent** | **S.a./Sta03** | **S.e/Sta03** | **S.a./Both** | **S.e./Both** |
| B | Control | Green | No Signal | Green | Red |
| C | PreservCyt Solution | Green | No Signal | Green | Red |
| D | SurePath Preservative | Green | No Signal | Green | Red |
| E | 50% Methanol | Green | No Signal | Green | Red |
| F | 50% Ethanol | Green | No Signal | Green | Red |
| G | 50% Reagent Alcohol | Green | No Signal | Green | Red |

With reference to Table (2), the table displays the presence and color of the fluorescent signal seen under each experimental condition, when tested with either *Staphylococcus aureus,* or the *Staphylococcus epidermidis* cells. In row A of Table (2), columns 2 through 5, the bacteria species and probe(s) are described in abbreviated format where "S.a./Sta03" indicates the Sta03 probe hybridized to *Staphylococcus aureus* in the various reagents, "Both" means both probes were present.

With reference to Table (2), column 2, all hybridization reagents tested with the Sta03 probe caused *Staphylococcus aureus* cells to fluoresce green under the microscope. All reagents resulted in similarly bright green fluorescence though there were subtle differences in brightness. Column 3 of the same table shows that there was no fluorescent signal seen with the *Staphylococcus epidermidis* cells when probed with the Sta03 probe. These results indicate that the Sta03 probe is able to penetrate the cell wall and subsequently bind specifically to the target sequence in a slide based format using all of the reagents tested. The fact that the *Staphylococcus epidermidis* cells did not become fluorescent green indicate that under the conditions tested the various reagents supplied sufficient stringency to only allow specific hybrid formation.

With reference to columns 4 and 5 of Table (2), all cells were made to fluoresce either red or green when both probes were added to the hybridization reaction. These data combined with the data in columns 2 and 3 demonstrate that all of the reagents are able to provide conditions suitable for specific hybridization, and that both species of bacteria were available to hybridization.

It is therefore concluded that PNA probes hybridize specifically to target nucleic acid in aqueous alcohol solutions, such as 50% (v/v) ethanol, 50% (v/v) methanol, 50% (v/v) reagent alcohol, PreservCyt (45-55% methanol) and SurePath (15-20% ethanol, < 1% isopropanol, < 1% methanol) solutions.

### Example 3 - Combined fixation and hybridization in aqueous alcohol solution containing PNA probe

An experiment was performed to simulate the work flow in which a clinical sample might be processed. The experiment included use of PreservCyt Reagent as a combination fixative/hybridization solution (Fix/Hyb) which also contained a PNA probe for detection of a pathogen of clinical interest. Two closely related yeast species, both of which are commonly encountered in clinical settings and difficult to distinguish using standard methods were, included in the experiment.

Liquid cultures were prepared for *Candida albicans* (ATCC# 10231) and *Candida dubliniensis* (ATCC# Y17841). Fix/Hyb buffers were prepared containing either 20 nM of a *Candida albicans* specific PNA probe, Can03, or 20 nM of a eukaryote specific PNA probe, EuUni1.
Can03: Flu-OO-AGA GAG CAG CAT GCA (Rigby et al. J. Clin. Microbiol. 40:2182-218(2002)
EuUni1: Cy3-OO-AC AGA CTT GCC CTC (Perry-O'Keefe et al., J. Appl. Microbiol. 90:180-189) (2001)

The Can03 probe detects a region of 26S ribosomal RNA which is unique to *Candida-albicans,* the EuUni1 probe detects a region of 18S ribosomal RNA which is common across most eukaryotes. The Cy3 label fluoresces red when properly excited. The Can03 probe is 15 bases in length, the sequence of which is complementary to a region of the *Candida albicans* rRNA, but has 4 mismatches to the *Candida dubliniensis* rRNA. Samples were prepared by addition of 5 uL of either cell culture into 495 uL of either Fix/Hyb solution. Once prepared, samples were mixed, and kept at room temperature for 1 hour (to simulate transportation to the lab) and subsequently incubated for 1 hour in a water bath at 55 °C. Cells were pelleted by centrifugation in a microcentrifuge, and supernatants were aspirated off. Cells were rinsed once by addition of 500 uL phosphate buffered saline solution (PBS; 7 mM Na2HPO4, 7 mM NaH2PO4, 130 mM NaCl), followed by brief vortexing. Again, cells were pelleted by centrifugation, supernatants were aspirated, and cells were resuspended in 50 uL of TE buffer (10 mM Tris pH 9.0, 1 mM EDTA). 5 uL of the cell suspensions were spotted onto ThinPrep slides (Cytyc Corp., Boxborough, MA), and allowed to air dry. One drop of Mounting. Solution (AdvanDx, Inc., Woburn, MA) was added to each slide, followed by application of a cover glass. Cells were visualized on an Olympus BX51 fluorescent microscope equipped with a 60X oil immersion objective and FITC/Texas Red dual band filter for simultaneous observation of fluorescence from fluorescein and Cy3.

Cells were scored using a fluorescence scale of 0 through 3. The intensity of fluorescence at the different values of the scale is as follows:
0 = No visible fluorescence
1 = Weak fluorescence
2 = Clear positive fluorescence
3 = Strong fluorescence

The difference in intensity between 1 and 3 is approximately 50 fold. Slides were scored as described below in Table (3).

**Table (3)**

| | 1 | 2 | 3 |
|---|---|---|---|
| A | **Candida Species/Probe** | **Green Signal** | **Red Signal** |
| B | C.albicans/Can03 | 3 | 0 |
| C | C.dublinensis/Can03 | 1 | 0 |
| D | C.albicans/EuUni1 | 0 | 2 |
| E | C.dublinensis/EuUni1 | 0 | 2 |

With reference to Table (3), column 2, rows B and C, we observed a marked difference in the amount of green fluorescence between *C.albicans* and *C.dubliniensis. C.albicans* gave a bright green signal in clear contrast with a dark background while *C.dubliniensis* gave a weak green/brown signal which was barely distinguishable against the black background. With reference to Table (#), column 3, rows D and E, we observed a similar amount of red fluorescent signal between the two yeast species when probed with the universal eukaryotic probe. Both yeast species gave a clear red, signal in contrast to a dark reddish background. This observation demonstrates that both yeast species were available for detection with PNA probes, and that they both contained approximately equal amounts of RNA targets.

The data in Table (3) demonstrate that an alcohol based solution can be used as a combined fixation-hybridization solution where the PNA probe is in the alcohol solution prior to addition of the sample for discrimination between closely related species of clinical relevance. As a result of the combined fixation-hybridization composition, the experiment described uses very few manipulations to achieve high signal, and high specificity with very low background signal.

### Example 4 - Comparison to DNA

An experiment was performed simultaneously to the one described above in Example 3 using the exact same protocol, with a different set of probes and bacterial cultures. In this case, *Staphylococcus aureus* (ATCC#6538) and *Staphylococcus epidermidis* (ATCC# 14490) were used, in combination with the probes described in Example 2 (Sta03-Flu and Sep01-TAM), and a third probe, made of DNA. The DNA probe sequence was obtained from a publication (Kempf et al. J. Clin. Micro. 38(2) 830-838, 2000 describing sets of DNA probes which are useful for species specific identification in FISH assays. The DNA probe was purchased from Applied Biosystems, and had the following sequence and labeling.
Sa DNA: 6FAM-GAA GCA AGC TTC TCG TCC G

Each probe was added to the PreservCyt reagent prior to inoculation with bacterial cells as described above. Each Staphylococcus species was tested with each probe. PNA Probes were used at 100 nM final concentration, the DNA probe was used at 500 nM. In a deviation from the protocol described above in Example 4, AdvanDx slides were used instead of ThinPrep slides. Also, slides were washed post hybridization as described above in Example 3, though at a lower temperature (55 °C).

Slides were scored as described below in Table (#) using the same scale as described in Example 4.

**Table (4)**

| | 1 | 2 | 3 |
|---|---|---|---|
| A | **Staphylococcus Species/Probe** | **Green Signal** | **Red Signal** |
| B | S.aureus/ Sta03 | 2 | 0 |
| C | S.aureus/ Sep01 | 0 | 0 |
| D | S.aureus/ Sa DNA | 0 | 0 |
| E | S.epidermidis/ Sta03 | 0 | 0 |
| F | S.epidermidis / Sep01 | 0 | 2 |
| G | S.epidermidis / Sa DNA | 0 | 0 |

These data demonstrate specific detection of Staphylococcus species using PNA probes in a combination fixation/hybridization reagent. Neither the Sta03, nor the Sep01 probe had any cross hybridization. There was no detectable fluorescent signal from either species of bacteria probed with the DNA probe, though there was a general greenish background haze on the slides where it was used. It was not entirely surprising that the DNA probe did not work in this assay format since it is widely understood that alcohols inhibit the formation of DNA:DNA and DNA:RNA hybrids. This experiment provides further evidence that the hybridization properties of PNA can not be inferred from prior examples in which DNA was used.

### Example 5 - Hybridization in various concentrations of methanol

An experiment was performed to examine the effects of methanol concentration in the hybridization buffer. The assay protocol was the same as in Experiment 2 with a few exceptions. Staphylococcus aureus (ATCC# 6538) and Staphylococcus epidermidis (ATCC# 14990) cell lines were grown over night in an appropriate liquid medium. 7 mL of each cell culture was pelleted by centrifugation and supernatants were aspirated off. Each cell pellet was washed by resuspension in 25 mL PBS, followed by brief vortexing. Cells were pelleted again and supernatants were removed. Cell pellets were resuspended in 15 mL PreservCyt reagent to fix the cells. After 1 hour at room temperature, cell suspensions were stored at -20 °C until use. Hybridization solutions were prepared which contained either 20%, 30%, 40%, 50%, 60%, or 70% methanol in water and 250 nM of the Sta03 PNA probe. 250 uL of PreservCyt fixed cells were pelleted by centrifugation, and supernatants were removed. Cell pellets were resuspended in 50 uL of one of the methanol hybridization solutions and briefly vortexed. Cell suspensions were incubated at 55 °C for 30 minutes to allow hybridization of the PNA probe. Post hybridization, cells were pelleted, supernatants removed, and pellets were washed to remove free probe by suspension in 250 uL PBS. Cells were pelleted again, supernatants removed and the pellet was resuspended in 100 uL TE buffer. 5 uL of the TE suspensions were spotted onto microscope slides (AdvanDx, Woburn, MA), and allowed to air dry to completion. Slides were washed post hybridization as described above in Example 2, though at a lower temperature (55 °C). Following the wash, slides were air dried, then one drop of Mounting Solution (AdvanDx, Inc., Woburn, MA) was added to each slide, followed by application of a cover glass. Cells were visualized as before.

Slides were scored as displayed below in Table (5) using the same scale as described in Example 4.

**Table (5)**

| | 1 | 2 | 3 |
|---|---|---|---|
| A | **[Methanol]** | **S. aureus** | **S. epidermidis** |
| B | 20% | 1 | 0 |
| C | 30% | 1 | 0 |
| D | 40% | 2 | 0 |
| E | 50% | 3 | 0 |
| F | 60% | 3 | 0 |
| G | 70% | 3 | 2 |

With reference to Table (5) column 2, rows B-G, a clear trend was observed between methanol concentration and fluorescence in *S*. *aureus* cells. At 20% to 30% methanol very little fluorescence was generated, and the signal was somewhat mottled. At 40% methanol, the fluorescent signal increased significantly and was clear and bright. The 40% methanol sample is a true positive, whereas; the samples at 20 and 30 percent methanol were borderline positives. At 50% through 70% methanol, the signal became very bright. The cells in the 50% through 70% samples were bright green and in stark contrast to the dark background. At 70% methanol the cells became somewhat degraded in appearance, and were forming large clumps.

With reference to column 3, rows B-F of Table (5), the *S.epidermidis* cells did not display any significant fluorescent signal from 20% through 60% methanol. Though the cells in these samples were observed to be very weakly green, none of them were even borderline positive. At 70% methanol, there was sudden jump in fluorescent signal, accompanied by a similar change in cellular appearance as was seen with the *S.aureus* cells. It is not clear what happened to the 70% methanol samples, but it is obvious that in this particular assay format 70% methanol was not a particularly useful hybridization reagent (though there was a distinct difference in brightness between *S.aureus* and *S.epidermidis*)*.* It remains possible that 70% methanol or greater could be a useful hybridization reagent in other assay formats or that other alcohols may be used at 70% or higher concentrations.

### Example 6-Comparison with published fixation and hybridization reagents.

An experiment was performed to compare existing fixation and hybridization reagents with alcohol reagents. An exemplary method was taken from a publication in which PNA probes were demonstrated to be highly effective in "in solution" FISH assays (O'Keefe, et al., Environmental Molecular . Microbiology: Protocols and Applications. 207-217 (2001) ).

A liquid culture was prepared for *Candida albicans* (ATCC# Y12983). 5 mL of cells were pelleted by centrifugation in three separate tubes, and washed once with 5 mL of PBS. Supernatants were aspirated, and cells were resuspended in either of three fixation solutions, PreservCyt Reagent, SurePath Preservative Fluid, or 4% paraformaldehyde (4% paraformaldehyde in PBS adjusted to pH 8.0 with NaOH, and filter sterilized). After 1 hour at room temperature, cells were stored at 0 °C over night. The 4% paraformaldehyde fixed cells were pelleted, supernatants were aspirated, and cells were resuspended in 50% ethanol and held at room temperature for 30 minutes. 100 uL of each cell fixation suspension was pelleted by centrifugation, and resuspended in 50 uL of hybridization solution. The hybridization solutions tested were PreservCyt Reagent, SurePath Preservative Fluid, and solution hybridization buffer (SHB, 25 mM Tris-HCl, pH 9.0, 0.1M NaCl, 0.5% SDS) as was prepared in the forementioned reference. Each hybridization reagent contained 500 nM of the Can03 probe described in Example 3. Hybridizations were performed at 55 °C for 30 minutes. Cells were pelleted and washed once with 100 uL PBS. Cells were resuspended in 50 uL TE, and 10 uL of each suspension was spotted onto a microscope slide and allowed to air dry. Slides were washed and visualized as described in Example 5,

Slides were scored as displayed below in Table (6) using the same scale as described in Example 3.

**Table (6)**

| | **1** | **2** | **3** |
|---|---|---|---|
| **A** | **Fixation** | **Hybridization** | **Green Signal** |
| B | 4% Paraformaldehyde | SHB | 3 |
| c | PreservCyt | PreservCyt | 3 |
| D | PreservCyt | SurePath, | 3 |
| E | SurePath | SurePath | 2 |

With reference to Table (6), column 3 it is clear from the data that alcohol solutions can be used to obtain comparable results to exemplary fixation and hybridization methods. In rows B-D, the cells displayed very strong fluorescence regardless of hybridization buffer, or fixation buffer. When the SurePath Preservative Fluid was used as both a fixation and a hybridization buffer, the fluorescent signal was weaker than the others, though still clearly positive.

The invention has been described with reference to preferred embodiments thereof. However, it will be appreciated that those skilled in the art, upon consideration of this disclosure, may make modifications and improvements within the scope of the invention.

## Claims

1. A method for analysis of a target DNA or RNA in cells in a sample, the method comprising:
a) contacting the cells with a composition comprising: at least one PNA probe that specifically binds a target DNA or RNA in an aqueous alcohol solution comprising from 40% (v/v) to 60% (v/v) alcohol, wherein the alcohol is methanol, ethanol, isopropanol, or a mixture thereof;
b) incubating the cells and PNA probe(s) under conditions that form a specific binding complex between the PNA probe and any target DNA or RNA; and
c) detecting any bound PNA probe as being indicative of the presence of the target DNA or RNA in the cells.

2. The method of Claim 1, wherein step (c) of the method further comprises detecting the bound PNA probe by flow cytometry.

3. The method of Claim 1 or Claim 2, wherein the method further comprises placing the cells onto a microscope slide.

4. The method of any one of Claims 1 to 3, wherein the method further comprises filtering the cells.

5. The method of any one of Claims 1 to 4, wherein the method further comprises removing any unbound PNA probe prior to the detection step (c).

6. The method of any one of Claims 1 to 5, wherein the sample comprises urine, blood, wounds, sputum, cervical swabs, gastric lavage, bronchial washings, biopsies, aspirates, expectorates, food, beverages, water, pharmaceutical products, personal care products, dairy products, or environmental samples, and cultures thereof.

7. The method of Claim 1, wherein the aqueous alcohol solution comprises 45-55% (v/v) methanol, 2-4% EDTA (w/v), and 6-8% (w/v) sodium acetate.

8. The method of any one of Claims 1 to 7, wherein the aqueous alcohol solution comprises 10-70% (v/v) formamide.

9. The method of Claim 1, wherein the PNA probe further comprises at least one detectable label.

10. The method of Claim 9, wherein the detectable label(s) are selected from the group consisting of: a conjugate, a branched detection system, a chromophore, a fluorophore, a spin label, a radioisotope, an enzyme, a hapten, a lanthanide label, an acridinium ester and a luminescent compound.

11. The method of any one of Claims 1 to 10, wherein the at least one PNA probe specifically binds DNA or RNA of *Candida albicans, Candida dubliniensis, Staphylococcus aureus* or *Staphylococcus epidermidis.*

12. The method of Claim 1, wherein the PNA probe binds to a target sequence which is specific for a genetically based disease or is specific for a predisposition to a genetically based disease.

13. The method of Claim 1, wherein the PNA probe specifically binds DNA or mRNA of an oncogene.

14. The method of Claim 1, wherein the cells are from a microorganism optionally present in the sample.

15. The method of Claim 14, wherein the microorganism is *Staphylococcus aureus.*

16. The method of Claim 14, wherein the microorganism is *Candida albicans.*

17. The method of Claim 14, wherein the microorganism is human papillomavirus.

18. A method for analysis of a target nucleic acid in a sample, the method comprising:
(a) isolating and optionally amplifying nucleic acid;
(b) contacting the isolated and optionally amplified nucleic acid with at least one PNA probe in an aqueous alcohol solution comprising from 40% (v/v) to 60% (v/v) alcohol, wherein the probe specifically binds any target nucleic acid therein; and
(c) detecting the bound PNA probe as being indicative of the presence of the target nucleic acid in the sample.

19. A kit adapted to detect a target DNA or RNA in a sample, the kit comprising:
a) at least one PNA probe, that specifically binds the target DNA or RNA, provided in an aqueous alcohol solution comprising from 40% (v/v) to 60% (v/v) alcohol, wherein the alcohol is methanol, ethanol, isopropanol, or a mixture thereof ; and
b) directions for using the kit to perform a hybridization assay using PNA probes in an aqueous alcohol solution.

20. Use of at least one PNA probe that specifically binds a target DNA or RNA in an aqueous alcohol solution comprising from 40% (v/v) to 60% (v/v) alcohol, wherein the alcohol is methanol, ethanol, isopropanol, or a mixture thereof, for the analysis of a target DNA or RNA in a sample, wherein the sample comprises isolated and optionally amplified DNA or RNA.

## Patentansprüche

1. Verfahren zur Analyse einer Ziel-DNA oder -RNA in Zellen in einer Probe, umfassend die folgenden Stufen:
a) Inkontaktbringen der Zellen mit einer Zusammensetzung, die mindestens eine PNA-Sonde, die eine Ziel-DNA oder -RNA spezifisch bindet, in einer wässrigen Alkohollösung, die 40 % (V/V) bis 60 % (V/V) Alkohol umfasst, wobei der Alkohol Methanol, Ethanol, Isopropanol oder ein Gemisch derselben ist, umfasst;
b) Inkubieren der Zellen und der PNA-Sonde(n) unter Bedingungen zur Ausbildung eines Komplexes mit spezifischer Bindung zwischen der PNA-Sonde und einer etwaigen Ziel-DNA oder -RNA; und
c) Detektieren einer etwaigen gebundenen PNA-Sonde, was auf das Vorhandensein der Ziel-DNA oder -RNA in den Zellen schließen lässt.

2. Verfahren nach Anspruch 1, wobei die Stufe (c) des Verfahrens ferner das Detektieren der gebundenen PNA-Sonde durch Durchflusszytometrie umfasst.

3. Verfahren nach Anspruch 1 oder Anspruch 2, wobei das Verfahren ferner das Platzieren der Zellen auf einen Mikroskop-Objektträger umfasst.

4. Verfahren nach einem der Ansprüche 1 bis 3, wobei das Verfahren ferner ein Filtrieren der Zellen umfasst.

5. Verfahren nach einem der Ansprüche 1 bis 4, wobei das Verfahren ferner das Entfernen einer etwaigen nichtgebundenen PNA-Sonde vor der Detektionsstufe (c) umfasst.

6. Verfahren nach einem der Ansprüche 1 bis 5, wobei die Probe Harn, Blut, Wunden, Sputum, Zervixabstriche, Magenspülung, Bronchusspülungen, Biopsien, Punktate, Expektorate, Nahrungsmittel, Getränke, Wasser, pharmazeutische Produkte, Körperpflegeprodukte, Milchprodukte oder Umweltproben und Kulturen derselben umfasst.

7. Verfahren nach Anspruch 1, wobei die wässrige Alkohollösung 45 - 55 % (V/V) Methanol, 2 - 4 % EDTA (Gew./V) und 6 - 8 % (Gew./V) Natriumacetat umfasst.

8. Verfahren nach einem der Ansprüche 1 bis 7, wobei die wässrige Alkohollösung 10 - 70 % (V/V) Formamid umfasst.

9. Verfahren nach Anspruch 1, wobei die PNA-Sonde ferner mindestens eine detektierbare Markierung umfasst.

10. Verfahren nach Anspruch 9, wobei die detektierbare(n) Markierung(en) aus der Gruppe von einem Konjugat, einem verzweigten Detektionssystem, einem Chromophor, einem Fluorophor, einer Spinmarkierung, einem Radioisotop, einem Enzym, einem Hapten, einer Lanthanoidmarkierung, einem Acridiniumester und einer lumineszierenden Verbindung ausgewählt sind.

11. Verfahren nach einem der Ansprüche 1 bis 10, wobei die mindestens eine PNA-Sonde DNA oder RNA von *Candida albicans, Candida dubliniensis, Staphylococcus aureus* oder *Staphylococcus epidermis* spezifisch bindet.

12. Verfahren nach Anspruch 1, wobei die PNA-Sonde an eine zielsequenz bindet, die spezifisch für eine genetisch bedingte Erkrankung ist oder spezifisch für eine Prädisposition für eine genetisch bedingte Erkrankung ist.

13. Verfahren nach Anspruch 1, wobei die PNA-Sonde DNA oder mRNA eines Oncogens spezifisch bindet.

14. Verfahren nach Anspruch 1, wobei die Zellen von einem optional in der Probe vorhandenen Mikroorganismus stammen.

15. Verfahren nach Anspruch 14, wobei der Mikroorganismus *Staphylococcus aureus* ist.

16. Verfahren nach Anspruch 14, wobei der Mikroorganismus *Candida albicans* ist.

17. Verfahren nach Anspruch 14, wobei der Mikroorganismus das humane Papillomavirus ist.

18. Verfahren zur Analyse einer Zielnucleinsäure in einer Probe, umfassend die folgenden Stufen:
(a) Isolieren und optionales Amplifizieren der Nucleinsäure;
(b) Inkontaktbringen der isolierten und optional amplifizierten Nucleinsäure mit mindestens einer PNA-Sonde in einer wässrigen Alkohollösung, die 40 % (V/V) bis 60 % (V/V) Alkohol umfasst, wobei die Sonde eine etwaige Zielnucleinsäure in dieser spezifisch bindet; und
(c) Detektieren der gebundenen PNA-Sonde, was auf das Vorhandensein der Zielnucleinsäure in der Probe schließen lässt.

19. Kit, das zur Detektion einer Ziel-DNA oder -RNA in einer Probe angepasst ist, umfassend:
a) mindestens eine PNA-Sonde, die die Ziel-DNA oder -RNA spezifisch bindet und die in einer wässrigen Alkohollösung, die 40 % (V/V) bis 60 % (V/V) Alkohol umfasst, wobei der Alkohol Methanol, Ethanol, Isopropanol oder ein Gemisch derselben ist, bereitgestellt ist; und
b) Anleitungen zur Verwendung des Kits zur Durchführung eines Hybridisierungsassays unter Verwendung von PNA-Sonden in einer wässrigen Alkohollösung.

20. Verwendung von mindestens einer PNA-Sonde, die eine ziel-DNA oder -RNA spezifisch bindet, in einer wässrigen Alkohollössung, die 40 % (V/V) bis 60 % (V/V) Alkohol umfasst, wobei der Alkohol Methanol, Ethanol, Isopropanol oder ein Gemisch derselben ist, zur Analyse einer ziel-DNA oder -RNA in einer Probe, wobei die Probe isolierte und optional amplifizierte DNA oder RNA umfasst.

## Revendications

1. Procédé pour analyser un ADN ou un ARN cible dans des cellules d'un échantillon, le procédé consistant à :
a) mettre les cellules en contact avec une composition comprenant : au moins une sonde PNA qui se lie spécifiquement à un ADN ou à un ARN cible dans une solution aqueuse alcoolique comprenant entre 40 % (v/v) et 60 % (v/v) d'alcool, dans laquelle l'alcool est du méthanol, de l'éthanol, de l'isopropanol, ou un mélange de ceux-ci ;
b) incuber les cellules et la(les) sonde(s) PNA dans des conditions qui forment un complexe de liaison spécifique entre la sonde PNA et un quelconque ADN ou ARN cible ; et
c) détecter une quelconque sonde PNA liée comme étant indicatrice de la présence de l'ADN ou de l'ARN cible dans les cellules.

2. Procédé selon la revendication 1, dans lequel l'étape (c) du procédé consiste en outre à détecter la sonde PNA liée par cytométrie en flux.

3. Procédé selon la revendication 1 ou la revendication 2, dans lequel le procédé consiste en outre à placer les cellules sur une lame de microscope.

4. Procédé selon l'une quelconque des revendications 1 à 3, dans lequel le procédé consiste en outre à filtrer les cellules.

5. Procédé selon l'une quelconque des revendications 1 à 4, dans lequel le procédé consiste en outre à éliminer une quelconque sonde PNA non liée avant l'étape de détection (c).

6. Procédé selon l'une quelconque des revendications 1 à 5, dans lequel l'échantillon comprend de l'urine, du sang, des lésions, un crachat, des frottis cervicaux, un lavage gastrique, des lavages bronchiques, des biopsies, des aspirats, des expectorations, des aliments, des boissons, de l'eau, des produits pharmaceutiques, des produits d'hygiène corporelle, des produits laitiers, ou des échantillons environnementaux, et des cultures de ceux-ci.

7. Procédé selon la revendication 1, dans lequel la solution aqueuse alcoolique comprend 45-55 % (v/v) de méthanol, 2-4 % d'EDTA (p/v), et 6-8 % (p/v) d'acétate de sodium.

8. Procédé selon l'une quelconque des revendications 1 à 7, dans lequel la solution aqueuse alcoolique comprend 10-70 % (v/v) de formamide.

9. Procédé selon la revendication 1, dans lequel la sonde PNA comprend en outre au moins un marqueur détectable.

10. Procédé selon la revendication 9, dans lequel le(s) marqueur(s) détectable(s) est(sont) sélectionné(s) dans le groupe consistant en : un conjugué, un système de détection ramifié, un chromophore, un fluorophore, un marqueur de spin, un radio-isotope, une enzyme, un haptène, un marqueur lanthanide, un ester d'acridinium et un composé luminescent.

11. Procédé selon l'une quelconque des revendications 1 à 10, dans lequel la au moins une sonde PNA se lie spécifiquement à l'ADN ou l'ARN de *Candida albicans, Candida dubliniensis, Staphylococcus aureus* ou *Staphylococcus epidermidis.*

12. Procédé selon la revendication 1, dans lequel la sonde PNA se lie à une séquence cible qui est spécifique d'une maladie génétique ou est spécifique d'une prédisposition à une maladie génétique.

13. Procédé selon la revendication 1, dans lequel la sonde PNA se lie spécifiquement à l'ADN ou à l'ARNm d'un oncogène.

14. Procédé selon la revendication 1, dans lequel les cellules sont issues d'un micro-organisme éventuellement présent dans l'échantillon.

15. Procédé selon la revendication 14, dans lequel le micro-organisme est *Staphylococcus aureus.*

16. Procédé selon la revendication 14, dans lequel le micro-organisme est *Candida albicans.*

17. Procédé selon la revendication 14, dans lequel le micro-organisme est un papillomavirus humain.

18. Procédé pour analyser un acide nucléique cible dans un échantillon, le procédé consistant à :
a) isoler et éventuellement amplifier l'acide nucléique ;
b) mettre l'acide nucléique isolé et éventuellement amplifié en contact avec au moins une sonde PNA dans une solution aqueuse alcoolique comprenant entre 40 % (v/v) et 60 % (v/v) d'alcool, où la sonde se lie spécifiquement à un quelconque acide nucléique cible dans celle-ci ; et
c) détecter la sonde PNA liée comme étant indicatrice de la présence de l'acide nucléique cible dans l'échantillon.

19. Kit adapté pour détecter un ADN ou un ARN cible dans un échantillon, le kit comprenant :
a) au moins une sonde PNA, qui se lie spécifiquement à l'ADN ou à l'ARN cible, mise à disposition dans une solution aqueuse alcoolique comprenant entre 40 % (v/v) et 60 % (v/v) d'alcool, dans laquelle l'alcool est du méthanol, de l'éthanol, de l'isopropanol, ou un mélange de ceux-ci ; et
b) des instructions pour utiliser le kit afin de réaliser un essai d'hybridation en utilisant les sondes PNA dans une solution aqueuse alcoolique.

20. Utilisation d'au moins une sonde PNA qui se lie spécifiquement à un ADN ou à un ARN cible dans une solution aqueuse alcoolique comprenant entre 40 % (v/v) et 60 % (v/v) d'alcool, dans laquelle l'alcool est du méthanol, de l'éthanol, de l'isopropanol, ou un mélange de ceux-ci, pour analyser un ADN ou un ARN cible dans un échantillon, dans laquelle l'échantillon comprend de l'ADN ou de l'ARN isolé et éventuellement amplifié.
